## Europäisches Patentamt
## European Patent Office
## Office européen des brevets

(19)

(11) Numéro de publication : **0 080 151**
**B1**

(12) **FASCICULE DE BREVET EUROPÉEN**

(45) Date de publication du fascicule du brevet :
30.12.86

(51) Int. Cl.⁴ : **A 41 B 13/02**

(21) Numéro de dépôt : **82110574.9**

(22) Date de dépôt : **16.11.82**

(54) **Couche-culotte à jeter équipée de dispositifs d'attache par adhésif.**

(30) Priorité : **25.11.81 FR 8122101**

(43) Date de publication de la demande :
**01.06.83 Bulletin 83/22**

(45) Mention de la délivrance du brevet :
**30.12.86 Bulletin 86/52**

(84) Etats contractants désignés :
**BE DE GB IT**

(56) Documents cités :
**FR-A- 2 087 805**
**FR-A- 2 259 551**
**FR-A- 2 301 187**
**US-A- 4 227 530**

(73) Titulaire : **BOUSSAC SAINT FRERES B.S.F. Société anonyme dite:**
**12, rue du Vieux Faubourg**
**F-59800 Lille (FR)**

(72) Inventeur : **Lucas, Gérard**
**2, avenue des Jonquilles**
**F-59116 Bousbecque (FR)**
Inventeur : **de Jonckheere, Raphael**
**797, domaine de la Vigne**
**F-59910 Bondus (FR)**

(74) Mandataire : **Casalonga, Axel et al**
**BUREAU D.A. CASALONGA OFFICE JOSSE & PETIT**
**Morassistrasse 8**
**D-8000 München 5 (DE)**

Jouve, 18, rue St-Denis, 75001 Paris, France

EP 0 080 151 B1

## Description

La présente invention se rapporte à une couche-culotte à jeter du type comprenant une mince feuille extérieure imperméable portant, sur sa face interne, un matelas absorbant et, de préférence, une feuille interne perméable recouvrant la face interne de la feuille imperméable et ledit matelas, la couche-culotte étant munie de deux dispositifs d'attache par adhésif.

Des dispositifs d'attache par adhésif pour des couches-culottes sont bien connus (voir par exemple brevets US 3 180 355, 3 630 201, 4 047 530, 4 049 001, 4 050 453 et brevet français n° 74 36 169). Chaque dispositif d'attache comprend généralement une languette adhésive fixée sur une partie de la feuille extérieure de la couche-culotte, notamment de la partie arrière, et venant se coller, lors de la fermeture de la couche-culotte, sur l'autre partie de la feuille extérieure, notamment sur la partie avant.

Il arrive fréquemment que l'on veuille rouvrir la couche-culotte, par exemple pour vérifier si l'enfant a mouillé le matelas absorbant, et la refermer si le matelas absorbant n'est pas encore mouillé. Le même problème de réouverture et de refermeture de la couche-culotte peut se présenter lorsqu'on veut rectifier la tenue de la couche-culotte, par exemple en vue de la resserrer à la taille. Or, il s'avère qu'une telle tentative d'ouverture sur une couche-culotte à jeter sur laquelle l'enveloppe extérieure est constituée par une feuille mince provoque, soit la déchirure de la languette adhésive soit l'arrachement de la zone de la feuille à laquelle adhère la languette adhésive, cette zone arrachée de la feuille restant collée sur la languette adhésive. Dans un cas comme dans l'autre, il n'est plus possible de refermer la couche-culotte qui est ainsi inutilisable, bien que le matelas absorbant puisse ne pas encore être mouillé.

Pour permettre une telle réouverture et refermeture d'une couche-culotte à jeter, on a déjà proposé des dispositifs d'attache par adhésif (brevet US n° 3 989 048 et 4 014 339) dont les languettes adhésives en forme de double rabat replié en U ou en Z comprennent deux parties recouvertes d'une couche d'adhésif, ces deux parties pouvant être utilisées successivement en vue d'une première fermeture et d'une seconde fermeture de la couche-culotte. Toutefois, ces languettes à double fermeture sont de structure relativement compliquée puisqu'elles impliquent l'utilisation d'un rabat à pliage multiple, portant en plusieurs endroits bien définis des couches d'adhésifs et des couches anti-adhésives. L'utilisation de ces dispositifs connus, notamment en vue de la seconde fermeture, ne présente pas non plus la simplicité requise pour ce genre d'articles et implique en particulier une déchirure du rabat en deux endroits bien déterminés.

On connaît par ailleurs un dispositif d'attache à utilisations multiples pour une couche-culotte à jeter (brevet US n° 4 034 752) comprenant plusieurs languettes adhésives individuelles qui sont toutes fixées de façon détachable sur une partie de la feuille extérieure recouverte d'une couche anti-adhésive ; pour chaque fermeture, on détache une languette et on la colle sur les deux parties de la feuille extérieure, devant être reliées entre elles.

Enfin, par les brevets US n° 3 999 546 et 4 049 001, on connaît des dispositifs d'attache par adhésif avec deux rabats ou languettes superposés. Ces dispositifs sont relativement épais et présentent surtout l'inconvénient que l'on risque, à la première fermeture, d'utiliser par mégarde le rabat ou la languette destiné à la seconde fermeture de sorte que le dispositif d'attache ne peut plus servir à une seconde fermeture.

La présente invention a pour objet une couche-culotte à jeter munie de dispositifs d'attache par adhésif permettant, après une première fermeture, une réouverture et une seconde fermeture de la couche-culotte, tout en étant d'une structure et d'une fabrication simples et d'une utilisation sûre et aisée.

Telle qu'elle est revendiquée, la couche-culotte à jeter comprend une mince feuille extérieure imperméable portant, sur sa face interne, un matelas absorbant ainsi que, de préférence, une feuille intérieure perméable recouvrant la face interne de la feuille extérieure imperméable et ledit matelas. La couche-culotte est munie, pour fixer l'une à l'autre les parties avant et arrière de ladite feuille imperméable, au moment de l'utilisation, de deux dispositifs d'attache. Chaque dispositif d'attache comprend : a) un élément d'accrochage fixé par une couche d'adhésif de façon non détachable sur le bord de la face interne d'une des parties de ladite feuille imperméable, au moins partiellement recouverte par la feuille intérieure perméable ; b) un élément d'ancrage fixé de façon non détachable par une couche d'adhésif sur la face externe de ladite partie de la feuille imperméable ; et c) un rabat de fixation présentant une première partie qui, avant utilisation, est fixée de façon détachable par une couche d'adhésif sensible à la pression sur ledit élément d'accrochage ; et une deuxième partie qui, avant utilisation, est fixée de façon détachable par une couche d'adhésif sensible à la pression sur ledit élément d'ancrage. Selon l'invention, l'élément d'ancrage présente des perforations définissant des zones de contact direct entre la couche d'adhésif du rabat et la face externe de la feuille imperméable, lesdites zones de contact ayant une faible étendue suivant une direction parallèle à la direction de détachement par pelage du rabat de l'élément d'ancrage de façon que la fixation de la deuxième partie du rabat sur l'élément d'ancrage soit réalisée avec une adhérence supérieure à la fixation de la première partie sur l'élément d'accrochage.

Grâce au fait que les deux parties du rabat sont avant utilisation, fixées de façon détachable, la première sur l'élément d'accrochage et la seconde sur l'élément d'ancrage, il est possible de procéder à une première fermeture en détachant la première partie du rabat de l'élément d'accrochage et en la fixant de façon non détachable sur l'autre partie de la feuille imperméable, la seconde partie du rabat restant fixée sur l'élément d'ancrage. Si l'on désire ensuite procéder à une réouverture suivie d'une refermeture, il suffit de détacher la seconde partie du rabat de l'élément d'ancrage et, ensuite, de fixer de nouveau cette seconde partie du rabat sur la première partie de la feuille, soit à l'endroit de l'élément d'ancrage, soit à un endroit différent.

Divers modes de réalisation sont possibles pour cet élément d'ancrage, dans le cadre de la présente invention.

Suivant un mode de réalisation, l'élément d'ancrage peut être constitué, tout comme l'élément d'accrochage, par un matériau de support en forme de feuille fixé par une couche d'adhésif de façon non détachable à ladite feuille extérieure et portant, sur sa face opposée, une couche anti-adhérence.

Il est avantageux que l'élément d'accrochage et l'élément d'ancrage soient réalisés d'une seule pièce, sous la forme d'un rabat chevauchant le bord de la feuille imperméable.

Pour faciliter le détachement du rabat de l'élément d'accrochage lors de la première utilisation et de l'élément d'ancrage lors de la seconde utilisation, il est avantageux que les deux parties du rabat soient repliées sur elles-mêmes à leur extrémité libre, de manière que ces extrémités repliées n'adhèrent pas à l'élément d'ancrage et à l'élément d'accrochage et peuvent être saisies sans difficulté en vue du détachement des parties correspondantes du rabat par pelage de l'élément d'accrochage et de l'élément d'ancrage, à la première et à la seconde utilisation.

En se référant au dessin schématique annexé, on va décrire ci-après plus en détail un mode de réalisation préféré de l'invention, donné à titre d'exemple illustratif et non limitatif ; sur les dessins :

la figure 1 est une vue en perspective d'une couche-culotte à jeter équipée de deux dispositifs d'attache par adhésif conformes à l'invention ;

la figure 2 est une coupe longitudinale d'un dispositif d'attache, avant utilisation ;

la figure 3 est une coupe longitudinale du dispositif, après la première fermeture ;

la figure 4 est une coupe longitudinale du dispositif, après la seconde fermeture.

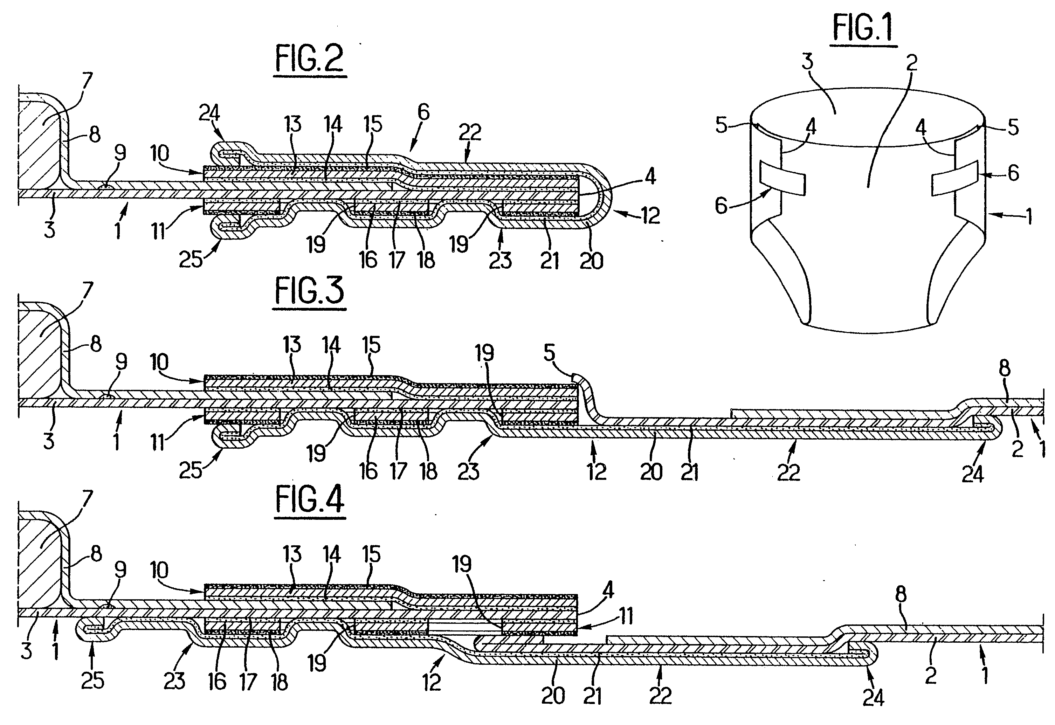

Il convient de noter que sur les fig. 2 à 4, les différents éléments constitutifs ont été illustrés, pour des raisons de clarté du dessin, avec des épaisseurs exagérées.

La couche-culotte à jeter illustrée par la fig. 1 comprend principalement une enveloppe constituée par une mince feuille extérieure 1 souple, imperméable, par exemple en polyéthylène. Cette feuille 1 de forme générale rectangulaire adopte, lorsqu'elle est posée sur l'enfant, la forme visible sur la fig. 1, avec une partie avant 2 et une partie arrière 3 dont les deux bords latéraux opposés 4 recouvrent les bords latéraux opposés 5 de la partie avant 2.

Pour pouvoir fermer la couche-culotte à hauteur de la taille de l'enfant, la partie arrière 3 comporte, sur chaque bord latéral 4, un dispositif d'attache par adhésif 6 coopérant avec la partie avant 2.

Sur la fig. 2 qui représente en coupe la zone d'un bord latéral 4 de la partie arrière 3 de la couche-culotte avec l'un des dispositifs d'attache 6, on reconnaît un matelas absorbant 7 posé sur la face interne de la feuille 1, ainsi qu'une feuille intérieure perméable 8, par exemple un voile de non-tissé, recouvrant le matelas 7 ainsi que la face interne de la feuille 1 à laquelle il est fixé par des lignes de colle 9.

Le dispositif d'attache 6 représenté sur la fig. 2 dans la position qu'il occupe avant l'utilisation comprend un élément d'accrochage 10, un élément d'ancrage 11 et un rabat de fixation 12.

L'élément d'accrochage 10 est constitué par un matériau 13 en forme de feuille muni sur l'une de ses faces d'une couche d'adhésif 14 et sur sa face opposée d'une couche anti-adhésive 15. L'élément d'accrochage 10 est fixé par la couche d'adhésif 14 sur la face interne de la partie 3 de la feuille imperméable 1, de manière à s'étendre depuis le bord latéral 4 en direction du matelas absorbant 7.

L'élément d'ancrage 11 est également constitué par un matériau 16 en feuille muni sur l'une de ses faces d'une couche d'adhésif 17 et sur sa face opposée d'une couche anti-adhésive 18. L'élément d'ancrage 11 est fixé par sa couche d'adhésif 17 sur la face extérieure de la partie avant 3 de la feuille 1, dans une position symétrique par rapport à l'élément d'accrochage 10.

L'élément d'ancrage 11 diffère de l'élément d'accrochage 10 par le fait qu'il présente plusieurs trous 19 qui le traverse de part en part de manière que la face extérieure de la feuille 1 se trouve mise à nue à l'endroit des trous 19.

Le rabat de fixation 12 est constitué par matériau de support 20 en forme de feuille muni sur l'une de ses faces d'une couche d'adhésif 21 sensible à la pression. Le rabat 12 qui présente une largeur égale ou de préférence inférieure à la largeur de l'élément d'accrochage 10 et de l'élément d'ancrage 11 et une longueur sensiblement égale à la somme des longueurs de l'élément d'accrochage 10 et de l'élément d'ancrage 11 est fixé à cheval sur le bord latéral 4 de la partie arrière 3 de la feuille 1 de manière que sa partie 22 soit appliquée par la couche d'adhésif 21 sensible à la pression sur l'élément d'accrochage 10 et que sa partie 23 soit appliquée par la couche d'adhésif 21 sur l'élément d'ancrage 11.

Du fait de la présence des trous 19 dans l'élément d'ancrage 11, la couche d'adhésif 21 de la partie 23 du rabat 12 vient se fixer, à l'endroit desdits trous 19, directement sur la face extérieure de la feuille imperméable 1 de la partie arrière 3 de la couche-culotte. De ce fait, la partie 23 du rabat 20 adhère plus fortement à la partie arrière 3 de la feuille 1 de la partie 22 appliquée sur la couche anti-adhésive 15 continue de l'élément d'accrochage 10.

L'extrémité libre de la partie 22 du rabat 12 est repliée sur elle-même en 24, de même que l'extrémité libre de la partie 23 du rabat 12 est repliée sur elle-même en 25. Ainsi, les deux extrémités libres 24 et 25 des parties 22 et 23 du rabat 12 n'adhèrent pas à l'élément d'accrochage 10 et à l'élément d'ancrage 11.

Pour fermer la couche-culotte à l'aide du dispositif d'attache 6, on saisit l'extrémité libre repliée 24 de la partie 22 du rabat 12 et on détache la partie 22 par pelage de l'élément d'accrochage 10. Cela n'implique qu'un faible effort dans la mesure où la couche d'adhésif 21 de la partie 22 du rabat n'adhère que modérément à la couche anti-adhésive 15 de l'élément d'accrochage 10. On fixe ensuite la partie 22 du rabat 12 détachée de l'élément d'accrochage 10, par sa couche d'adhésif 21 sur la face extérieure de la partie avant 2 de la feuille 1, le bord latéral 4 de la partie arrière 3 recouvrant le bord latéral 5 de la partie avant 2. Dans cette position illustrée par la fig. 3, la partie 22 du rabat 12 est fixée de façon non détachable par la couche d'adhésif 21 à la partie avant 2 de la feuille 1 et la partie 23 du rabat 12, bien qu'étant appliquée sur l'élément d'ancrage 11 recouvert d'une couche anti-adhésive 18, est fixée, grâce au contact direct de sa couche d'adhésif 21 avec la face extérieure de la feuille 1 à l'endroit des trous 19 de l'élément d'ancrage 11, avec une force suffisante à la partie avant 3 de la feuille 1 pour résister aux efforts que le dispositif d'attache subit pendant la fermeture et au cours de l'utilisation ultérieure.

Pour ouvrir de nouveau la couche-culotte, on saisit l'extrémité libre 25 de la partie 23 du rabat 12 et on détache la partie 23 du rabat par pelage de la partie arrière 3 de la feuille 1 c'est-à-dire de l'élément d'ancrage 11. Ce détachement est possible, sans destruction de la partie arrière 3 de la feuille 1, par le fait que les trous 19 de l'élément d'ancrage 10 sont suffisamment petits pour que la feuille 1 ne puisse pas être arrachée à l'endroit des trous 19 lors du détachement de la partie 23 du rabat 12. Par conséquent, la partie 23 du rabat 12, une fois détachée de l'élément d'ancrage 11, est intacte et peut être utilisée pour refermer la couche-culotte, par fixation de la partie 23 du rabat 12, dans la position voulue, sur la partie arrière 3 de la feuille 1. Sur la fig. 4, on a représenté cette seconde fermeture de la couche-culotte, la partie 23 du rabat 12 étant fixée sur la partie arrière 3 de la feuille 1 dans une position différente de celle dans laquelle la partie 23 était initialement fixée sur l'élément d'ancrage 11.

Bien entendu, si, à la seconde fermeture, on fixe la partie 23 du rabat 12 sur la partie arrière 3 de la feuille 1 exactement à l'endroit de l'élément d'ancrage 11, il est possible d'ouvrir une seconde fois et de refermer la couche-culotte. Par contre, si, à la seconde fermeture, la partie 23 du rabat 12 est collée sur la partie arrière 3 de la feuille 1 à un endroit différent, la partie 23 se trouve fixée de façon non détachable à la partie arrière 3 de la feuille 1, de sorte qu'une nouvelle ouverture de la couche culotte entraîne une déchirure du rabat 12 ou un arrachement de la feuille 1.

Des adhésifs sensibles à la pression pour la couche 21 du rabat 12 et des produits anti-adhésifs, par exemple à base de silicone, pour les couches 15 et 18 de l'élément d'accrochage 10 et de l'élément d'ancrage 11 sont bien connus par l'homme de l'art et leurs compositions n'entrent pas dans le cadre de la présente invention.

Bien entendu, le mode de réalisation décrit ci-dessus et représenté sur le dessin annexé n'a été donné qu'à titre illustratif et non limitatif et de nombreuses modifications et variantes sont possibles dans le cadre de l'invention.

Ainsi, en cas d'utilisation, pour les couches anti-adhésives 15 et 18 de l'élément d'accrochage 10 et de l'élément d'ancrage 11, d'un produit qui, tout en permettant un détachement aisé des parties 22 et 23 du rabat 12, assure néanmoins une adhérence suffisante de la couche d'adhésif 21 du rabat 12 pour supporter les efforts auxquels le rabat 12 est exposé pendant et après la fermeture de la couche-culotte, il est possible de réaliser de manière identique, c'est-à-dire sans les trous 19, l'élément d'accrochage 10 et l'élément d'ancrage 11.

Au lieu d'utiliser, pour l'élément d'accrochage 10 et l'élément d'ancrage 11, un matériau recouvert d'une couche anti-adhésive il serait également possible d'utiliser un matériau en feuille ayant une solidité suffisante pour résister à la déchirure et à l'arrachement. Les deux éléments 10, 11 pourraient être, par exemple, réalisés à partir du même matériau que la feuille 1, mais avec une épaisseur plus importante, ou également à partir d'un matériau en feuille différent, plus résistant, par exemple le polypropylène au lieu du polyéthylène utilisé pour la feuille 1.

Par ailleurs, il serait également possible de prévoir des trous 19, non seulement dans l'élément d'ancrage 11, mais également dans l'élément d'accrochage 10.

Dans le cas où l'élément d'accrochage 10 et l'élément d'ancrage 11 sont réalisés de façon identique, ces deux éléments peuvent être constitués d'une seule pièce chevauchant le bord 4 de la partie arrière 3 de la feuille 1.

L'invention s'applique à tous les types de couches-culottes, notamment avec ou sans élastique à la taille et/ou à l'entrejambe, avec matelas absorbant de toutes formes s'étendant jusqu'aux bords latéraux de la feuille extérieure où se terminant à distance de ces bords, avec ou sans échancrures pour les jambes, etc...

**Revendications**

1. Couche-culotte à jeter comprenant une mince feuille extérieure imperméable (1) portant, sur sa face interne un matelas absorbant (7) ainsi que, de préférence, une feuille intérieure perméable (8) recouvrant la face interne de la feuille extérieure imperméable (1) et ledit matelas (7) cette couche-culotte étant munie pour fixer l'une à l'autre les parties avant (2) et arrière (3) de ladite feuille imperméable, au moment de l'utilisation, de deux dispositifs d'attache, chaque dispositif d'attache comprenant :

a) un élément d'accrochage (10) fixé par une couche d'adhésif (14) de façon non détachable sur le bord de la face interne d'une des parties de ladite feuille imperméable (1), au moins partiellement recouverte par la feuille intérieure perméable (8)

b) un élément d'ancrage (11) fixé de façon non détachable par une couche d'adhésif (17) sur la face externe de ladite partie de la feuille imperméable (1) ; et

c) un rabat de fixation (12) présentant une première partie qui, avant utilisation, est fixée de façon détachable par une couche d'adhésif sensible à la pression sur ledit élément d'accrochage ; et une deuxième partie (23) qui, avant utilisation, est fixée de façon détachable par une couche d'adhésif (21) sensible à la pression sur ledit élément d'ancrage, caractérisé par le fait que l'élément d'ancrage présente des perforations définissant des zones de contact direct entre la couche d'adhésif du rabat et la face externe de la feuille imperméable, lesdites zones de contact ayant une faible étendue suivant une direction parallèle à la direction de détachement par pelage du rabat de l'élément d'ancrage de façon que la fixation de la deuxième partie (23) du rabat (12) sur l'élément d'ancrage (11) soit réalisée avec une adhérence supérieure à la fixation de la première partie (22) sur l'élément d'accrochage (10).

2. Couche-culotte suivant la revendication 1, caractérisée par le fait que ledit élément d'ancrage est constitué, tout comme ledit élément d'accrochage (10), par un matériau de support (16) en forme de feuille fixé par une couche d'adhésif (17) de façon non détachable à ladite feuille imperméable et portant, sur sa face opposée, une couche anti-adhésive (18).

3. Couche-culotte suivant l'une des revendications précédentes, caractérisée par le fait que les deux parties du rabat sont repliées sur elles-mêmes à leur extrémité libre (24, 25), de manière que les extrémités repliées n'adhèrent pas à l'élément d'ancrage et à l'élément d'accrochage.

## Claims

1. Disposable integral diaper comprising a thin impervious external sheet (1) carrying, on its inner face, an absorbent pad (7) and, preferably, a permeable internal sheet (8) covering the inner face of the external impervious sheet (1) and the said pad (7), this integral diaper being equipped for fixing the front (2) and rear (3) parts of the said impervious sheet to each other at the time of use, with two attachment devices, each attachment device comprising :

a) a hooking member (10) fixed in an unremovable manner by a layer of adhesive (14) to the edge of the inner face of one of the parts of the said impervious sheet (1), which is at least partly covered by the permeable internal sheet (8).

b) an anchoring member (11) fixed in an unremovable manner by a layer of adhesive (17) on the outer face of the said part of the impervious sheet (1), and

c) a fixing flap (12) having a first part which, before use is fixed in a removable manner, by a layer of pressure-sensitive adhesive on the said hooking member, and a second part (23) which, before use, is fixed in a removable manner by a layer of pressure-sensitive adhesive (21) on the said anchoring member characterized in that the anchoring member has perforations defining zones of direct contact between the adhesive layer of the flap and the outer face of the impervious sheet, the said contact zones being small in extent along a direction parallel to the direction in which the flap is detached by peeling from the anchoring member so that the fixing of the second part (23) of the flap (12) to the anchoring member (11) is produced with greater adhesion than the fixing of the first part (22) to the hooking member (10).

2. Integral diaper according to Claim 1, characterized in that the said anchoring member consists, as does the said hooking member (10), of a supporting material (16) in sheet form which is fixed in a unremovable manner by a layer of adhesive (17) to the said impervious sheet and which carries a release layer (18) on its opposite face.

3. Integral diaper according to either of the preceeding claims, characterized in that the two parts of the flap are folded back onto themselves at their free end (24, 25), so that the folded ends do not adhere to the anchoring member and to the hooking member.

## Patentansprüche

1. Wegwerf-Windelhöschen mit einer undurchlässigen dünnen Außenfolie (1), und mit einer Absorptionseinlage (7) an der Innenseite, sowie vorzugsweise einer durchlässigen Innenfolie, die die Innenseite der undurchlässigen Außenfolie und die Absorptionseinlage bedeckt, wobei dieses Windelhöschen mit zwei Befestigungvorrichtungen zum Befestigen des Vorder- (12) und Rückenteils (13) der genannten undurchlässigen Folie einander beim Gebrauch versehen ist, wobei jede Befestigungsvorrichtung enthält :

a) ein Haftelement (10), das mit einer Klebeschicht (14) in nicht lösbarer Weise am Rand der Innenseite des einen Teils der genannten undurchlässigen Folie (1) befestigt ist, die wenigstens teilweise von der undurchlässigen Innenfolie (8) bedeckt ist ;

b) ein Verankerüngselement (11), das in nicht lösbarer Weise mit einer Klebeschicht (17) auf der Außenseite der genannten undurchlässigen Folie (1) befestigt ist ; und

c) eine Befestigungsklappe (12) mit einem ersten Teil, der vor Gebrauch auf lösbare Weise mit einer druckempfindlichen Klebeschicht auf das genannte Haftelement befestigt ist, sowie einem zweiten Teil (23), der vor Gebrauch auf lösbare Weise mit einer druckempfindlichen Klebeschicht (21) auf das genannte Verankerungselement befestigt ist, dadurch gekennzeichnet, daß das genannte Verankerungselement Perforationen aufweist, die Direktkontaktzonen zwischen der Klebeschicht der Klappe und der Außenfläche der undurchlässigen Folie definieren, wobei die genannten Kontaktzonen in der Richtung parallel zur Richtung des Aufreißens durch Abziehen des Verankerungselements nur eine kleine Ausdehnung haben, so daß die Befestigung des zweiten Teils (23) der Klappe (12) auf dem Verankerungselement (11) mit einer stärkeren Haftung realisiert wird als die Befestigung des ersten Teils (22) auf dem Haftelement (10).

2. Windelhöschen gemäß Anspruch 1, dadurch gekennzeichnet, daß das genannte Verankerungselement ebenso wie das genannte Haftelement (10) aus einem Trägermaterial (16) in der Form einer durch eine Klebeschicht (17) auf nicht lösbare Weise an der genannten undurchlässigen Folie befestigt ist und auf seiner gegenüberliegenden Seite eine sogenannte release Schicht (18) trägt.

3. Windelhöschen gemäß einem beliebigen der vorstehenden Ansprüche, dadurch gekennzeichnet, daß die zwei Teile der Klappe an ihren freien Enden umgeschlagen sind, so daß die umgeschlagenen Enden nicht am Verankerungselement und am Haftelement haften.

FIG.1

FIG.2

FIG.3

FIG.4

0 080 151